# EUROPEAN PATENT SPECIFICATION

(11) **EP 1 429 623 B1**
(45) Date of publication and mention of the grant of the patent: **21.10.2009**
(21) Application number: 02766118.0
(22) Date of filing: 27.08.2002
(51) Int. Cl.: A23K 1/18, A01K 67/00, A01H 1/00

(54) **DELIVERY OF DISEASE CONTROL IN AQUACULTURE AND AGRICULTURE USING NUTRITIONAL FEEDS CONTAINING BIOACTIVE PROTEINS PRODUCED BY VIRUSES**
ABGABE VON KRANKHEITSBEKÄMPFUNG IN AQUAKULTUR UND LANDWIRTSCHAFT UNTER VERWENDUNG VON NÄHRSTOFFUTTERMITTELN, DIE VON VIREN PRODUZIERTE BIOLOGISCH AKTIVE PROTEINE ENTHALTEN
APPORT DE PRODUITS DE LUTTE CONTRE LES MALADIES EN AQUACULTURE ET AGRICULTURE AU MOYEN D'ALIMENTS NUTRITIONNELS CONTENANT DES PROTEINES BIOACTIVES PRODUITES PAR DES VIRUS

(30) Priority: 27.08.2001 US 314637 P
(43) Date of publication of application: 23.06.2004
(73) Proprietor: Advanced Bionutrition Corporation, Columbia, MD 21045 (US)
(72) Inventor: KYLE, David, J., Catonsville, Maryland 21228 (US)
(74) Representative: Warcoin, Jacques
(86) International application number: PCT/US2002/027198
(87) International publication number: WO 2003/017780

(56) References cited:
- US-A- 3 889 007
- US-A- 5 202 422
- US-A- 5 618 574
- US-A- 5 863 775
- MASON H.S. ET AL.: 'Expression of Norwalk virus capsid protein in transgenic tobacco and potato and its oral immunogenicity in mice' PROC. NATL. ACAD. SCI. USA vol. 93, May 1996, pages 5335 - 5340, XP002025358
- MODELSKA A. ET AL.: 'Immunization against rabies with plant derived antigen' PROC. NATL. ACAD. SCI. USA vol. 95, March 1998, pages 2481 - 2485, XP002921640
- YU ET AL.: 'A plant-based multicomponent vaccine protects mice from enteric disease' NATURE BIOTECH. vol. 19, June 2001, pages 548 - 552, XP002960082
- ARAKAWA ET AL.: 'Plants are not just passive creatures' NATURE MEDICINE vol. 4, May 1998, pages 550 - 551, XP002960083

## Description

### BACKGROUND OF THE INVENTION

### Field of the Invention

This invention is directed to the use of edible materials which are used as feed components in aquaculture or agriculture, and which also contain exogenous peptides and/or antibodies or antibody fragments which will convey resistance or immunity to viral or bacterial pathogens or otherwise improve the health and performance of the species consuming said edible materials. In particular, the exogenous peptides, proteins, and/or antibodies or antibody fragments are produced by cellular material infected by a virus that codes for the production of said peptides and/or antibodies or antibody fragments.

### Related Art

Certain plant products have been produced using specific genetic modification to express proteins and/or antibodies of therapeutic value. The group at the Boyce Thompson Institute at Cornell have cloned a viral coat protein into bananas so that when ingested by humans, this will be equivalent to delivering an oral vaccine to those humans. This concept has not been extended to microbes.

There are several plant biotech companies such as Meristem, Large Scale Biology, and Prodigene, which are now expressing certain human therapeutic proteins in the plants including antibodies. Large Scale Biology is expressing proteins in tobacco plants using a tobacco mosaic virus as a vector to produce the protein of interest. The protein is then isolated and purified from the plant material and used for human therapeutic purposes. In this way the plant genome itself is actually not modified, but rather the genome of the infecting virus carries the gene of interest.

Recombinant microbes including bacteria, yeast and fungi have been used to produce human therapeutic proteins. However, such recombinant microbes have not been used for agricultural purposes incorporating ingestion of the whole organism. In both the plant and microbial cases, the recombinant organism has simply been used as a factory, and the therapeutic protein is then isolated and purified prior to use.

Certain plant products have been produced which contain proteins and/or antibodies of therapeutic value by infecting the plant with a virus that expresses the protein of interest. Large Scale Biology has a series of patents protecting this technology but the purpose is to produce purified proteins for pharmaceutical purposes which requires an extensive purification procedure following harvesting of the plant material. These patents do not involve the use of the intact plant material as a source of both nutrition and disease control except under the unusual condition that the pharmaceutical product is expressed in the fruit of the plant.

Certain recombinant proteins have been produced in insect cells using an insect virus expression system (Baculovirus). Such proteins are also produced in intact insect larvae following infection with modified Baculoviruses. In both cases, the insect cells or larvae are used as factories to produce the protein of interest, and the recombinant protein is then purified for pharmaceutical purposes. Insect cells or larvae infected with baculovirus may be particularly useful in the expression of certain human therapeutic proteins because the post-translational modifications of the therapeutic proteins are similar to the post-translational modifications imparted upon expression in human cells.

The Sindbis arbovirus was used to deliver high levels of gene expression *in vivo* in non-host arthropod species without causing cytopathic effects in infected cells or impairing the development of the organism. A replication competent Sindbis virus containing the coding region of green fluorescent protein (GFP) produced productive infections when injected into insect larvae and pupae (Lewis, *et al*., 1999). Thus, virus-mediated ectopic gene expression has been accomplished in arthropods, a phylum that includes the classes Crustacea and Insecta.

Antibiotic doping is used routinely in the aquaculture setting. Typically, the pure or semipure antibiotics are added directly to the water column or feeding system; however, crude fermentation broths, particularly broths including cells, have not been used for any kind of therapeutic delivery system.

Production of amino acids such as lysine typically involves a genetically modified microorganism which overproduces the amino acid of interest and excretes it into the fermentation medium. The wastestream from such a fermentation would include biomass containing the amino acid, and this wastestream product could be used as a crude delivery form of the small molecule nutritive amino acid.

A Baculovirus expression system is an efficient method for expressing proteins in insect cell culture. Baculovirus is in the family Baculoviridae, a diverse group of large double stranded DNA viruses that infect arthropods, including insects, arachnids, and crustaceans. Baculoviruses are species-specific and do not infect vertebrates nor propagate in mammalian cells in culture.

### SUMMARY OF THE INVENTION

The present invention provides for a composition of matter (the feed) and the use of this feed for the manufacture of a medicament for the delivery of a therapeutic dose of a bioactive peptide or protein.

In one embodiment, this invention provides an aquaculture or an agriculture feed containing plant biomass comprising one or more proteins, antibodies, or a combination thereof, where the proteins and antibodies are non-native to the plants. Preferably, the host plants are selected from tobacco, corn, soybean, canola, sunflower, or any other cultivated crop. The plant genome itself may be modified to express the proteins or antibodies or antibody fragments. The plants may be infected with a virus or viruses which encode the proteins or antibodies or antibody fragments recombinantly. While in some cases the host and expressed protein may be consumed together without further processing, preferably the plant material, not necessarily the fruit, would be modified in some way to make the material edible to non-human animals. Such a modification may include, but not be limited to, homogenizing, cooking, baking, extruding, solubilizing, or treatment with enzymes.

In another embodiment, this invention provides an aquaculture or an agriculture feed containing insect biomass comprising one or more proteins, antibodies, or a combination thereof, where the proteins and antibodies are non-native to the insects. Preferably, the insects are larval stages of lepidoptera. The insect genome itself may be modified to express the proteins or antibodies or antibody fragments. The insects may be infected with a virus or viruses which encode the proteins or antibodies or antibody fragments and upon infection is expressed recombinantly. In a preferred mode, the insect material would be modified in some way to make the material edible to non-human animals. Such a modification may include, but not be limited to, homogenizing, cooking, baking, extruding, solubilizing, or treatment with enzymes. This invention contemplates the use of the whole insect larvae or a portion thereof as a feed additive. This invention also contemplates the use of the larvae along with its entire larval cultivation matrix, as all these materials may convey feed materials themselves. Such a larvae will typically contain the protein or proteins of interest but the purification steps are not necessary for its use in animal feeds.

In another embodiment, this invention provides a method of delivering therapeutic proteins to a non-human animal comprising administering to a non-human animal a feed comprising a plant or insect expressing a non-native therapeutic protein. This method is particularly suitable for the non-human animal subjected to intensive agricultural practices, or for fish or shellfish in aquaculture. In a preferred mode, the therapeutic protein or proteins is (are) recombinant protein(s) expressed directly by the plant or insect. The plant or insect is infected by a recombinant virus which expresses the therapeutic protein recombinantly. Preferred therapeutic proteins include a protein or proteins which inhibit(s) the growth or replication of a pathogen such as *Vibrio* species, or a protein or proteins which inhibit(s) shrimp viruses such as, but not limited to, Taura or White spot virus infection in shrimp, or a recombinantly expressed antibody or antibody fragments to viruses, or a protein which, when introduced orally to an animal will immunize said animal as in the case of an oral vaccine.

In another, preferred, embodiment, this invention provides a method of transfecting crustaceans with non-native therapeutic proteins using baculovirus. This method is particularly suitable for crustaceans in aquaculture. Preferably, the crustaceans are Pacific white shrimp (*Penaeus vannmei*) and the baculovirus is Autographa californica nuclear polyhedrosis virus (AcNPV). The crustacean may be infected either by injection or orally, by incorporating the virus into the crustacean's food. The baculovirus may be engineered to express green fluorescent protein (GFP) for monitoring infection.

### DETAILED DESCRIPTION OF THE EMBODIMENTS

The marine environment is filled with bacteria and viruses that can attack fish and/or shellfish. Infection by such bacteria or viruses can devastate intensive marine-based farms very quickly. The same is true for terrestrial environments where viral or bacterial infections can also dramatically limit farm productivity. One of the major disease control problems in shrimp aquaculture today is infection by certain viruses (e.g., White Spot, Taura, etc.). Neither antibiotic, nor probiotic strategies will work in this situation, and shrimp cannot be vaccinated by methods analogous to those used for fish. Shrimp, like all crustaceans have only a rudimentary immune system so they are particularly susceptible to devastation by viral attacks.

This invention provides a solution to this problem by providing a nutritional control method using the target animal's feed as the vector to deliver anti-viral antibodies or fragments thereof, directly to the shrimp. These "designer feeds" would deliver a therapeutic dose of antibody directly to the gut of the shrimp. This approach is known as "passive immunity" because the antibody remains outside the host organism and simply prevents viral infestation through the gut wall. The invention envisions the use of transgenic multicellular organisms (plants, animals, insects, etc) to deliver the antibody to the gut of the target animal through the consumption of the transgenic multicellular organism. Alternatively, the feed source itself may be infected with a host-specific virus that is engineered to produce the antibody, or fragment thereof, of interest in a multicellular organism that can be fed to the target animal. Alternatively, the feed material may deliver a portion of the virus (*e*.*g*. a coat protein) or fragment thereof in order to actively immunize the shrimp, other shellfish or finfish or terrestrial animal that consumes the feed.

This invention provides advantages in production and delivery of the antimicrobial compounds or antibodies by packaging them in a plant or insect source compared to a fermentative source. Sterile fermentation is important for Drug-GMP compliance whereas the less-pure sources from plants or insects will be perfectly fine for animal use. Plants and insects are common elements in the food chain for either aquatic species or terrestrial species (Aquaculture or Agriculture). A second value in eukaryotic processing (e.g., by plants or insect cells) is that the post-translational modifications undertaken by these species may be more "native" compared to recombinant products from bacteria. One of the major problems with bacterial production of human proteins is that the microbially produced recombinant proteins are ineffective because of incorrect post-translation modifications.

AcNPV is a Baculovirus commonly used in laboratory protein expression. It is shed from cells during early stages of infection by budding of the cell membrane; in later stages of infection, viruses are encased in intracellular occlusion bodies, which are large protein crystals. AcNPV is commonly used in the laboratory to infect insect cell culture lines. Vectors and molecular biology supplies, and methods for Baculovirus expression vector systems, including AcNPV, are readily available from commercial suppliers.

Antibodies or antibody fragments to desired targets, such as White Spot virus or Taura virus, may be prepared by routine immunization and selection of monoclonal antibody producing hybridomas, or by screening viral or bacterial expression libraries of immunoglobulin genes and gene fragments (Collagen, *et al*.). Nucleic acid sequences encoding the binding sites of the selected antibodies can be cloned using standard methods (Ausubel, *et al*.) and antibodies may be expressed from recombinant plants, animals or insects or cloned into viruses that infect the desired feed materials.

There are a number of well known bactericidal and bacteriostatic peptides which will inhibit microbial growth and include, but are not limited to cecropins, peneadins, bactenecins, callinectins, myticins, tachyplesins, clavanins, misgurins, pleurocidins, parasins, histones, acidic proteins, and lysozymes. These peptides may be made in a plant material such as tobacco, soybean, corn, sunflower, cotton, safflower, canola or any other agronomic species using recombinant methods well known to those in the art, and thus provided as a feed component to convey resistance or tolerance to infestation. Suitable plant material also includes macroalgae (Kelps) which are grown worldwide as a commodity feed crop in aquaculture. Macroalgae are the foodstuffs of many aquaculture species, and this invention contemplates recombinant production of therapeutic proteins in the natural or farm diet of juvenile fish (e.g., half-grown catfish) as well as fish larva. Thus, within the contemplation of this invention are macroalgae or insects or other host organism that make up part of the food chain for the feeding of larvae, juveniles and adults in aquaculture, as well as the same life sequence in the terrestrial animal feeds (e.g. pigs, chickens, and cows).

Edible materials can be any materials that are ingested. Preferably, edible materials may be of plant or animal (vertebrate or invertebrate) origin. Edible materials may be the whole plant or animal or any parts thereof. One embodiment of this invention would be where the plant or animal is genetically modified to produce the exogenous peptide and/or antibody or antibody fragments directly.

Post harvest processing of some sort may be required to prepare the material for use as feeds. This invention contemplates normal (known) processes for converting the insect or plant material into feeds. Such normal process include homogenization followed by extrusion into pellets of various sizes depending on the application (e.g., larval, juvenile or adult). Other modes of preparation would include spray drying, fluid bed drying, or even providing the material as a liquid suspension.

By "patient" it is meant any living animal, including, but not limited to, a human who has, or is suspected of having or being susceptible to, a pathologic condition, disease, or disorder, or who otherwise would be a subject of investigation relevant to a pathologic condition, disease, or disorder. Accordingly, a patient can be an animal that has been bred or engineered as a model for any pathologic condition, disease, or disorder. Likewise it can be a human suffering from, or at risk of developing, a pathologic condition, disease, or disorder. Similarly, a patient can be an animal (such as a farm animal, a dairy animal, a ranch animal, an animal that lives under water, or an animal cultivated on land or in water for food or other commercial use, an experimental animal, or a pet animal) including a human, who is serving as a healthy control for investigations into pathologic conditions, diseases, or disorders.

### EXAMPLES

The invention as contemplated herein, is described in the following examples, but its utility is not limited to the examples provided.

**Example 1. Incorporation of an antibody into a plant-based feed.** A particular viral or bacterial pathogen is chosen and used to prepare monoclonal antibodies using procedures described in "Current Protocols in Immunology" or other procedures known to those skilled in this field. The white spot virus, for example, contains three major coat proteins, and antibodies or antibody fragments can be prepared to any or all of these proteins. Gene(s) coding for this antibody or an appropriate antibody fragment (Fab) are isolated and amplified in an appropriate vector. The gene is spliced into a transformation vector suitable for plant transformation. The transformation vector is chosen so that the antibody will be overexpressed in the plant cellular biomass. The vector may be targeted to the edible portion of the plant (*i*.*e*., seeds) so that normal harvesting methodologies can be used. Alternatively, the vector may be targeted to the unused portion of the plant (stems and leaves) so that these less valued materials can be used as value added components to the crop plant without affecting the yield or quality of the normally harvested portion. The biomass in which the antibody is expressed is then used as a feed additive in such a way as to provide the antibody or antibody fragment directly to the animal, thus providing passive immunity.

**Example 2. Expression of a bactericidal or bacteriostatic protein in a plant-based feed.** A bactericidal or bacteriostatic protein is chosen for the particular application. For example, proteins of the penaeidin class may be chosen for pathogenic control in shrimp. Penaeidins are members of a family of antimicrobial peptides isolated from crustaceans (*e*.*g*., Penaeus shrimp). Antimicrobial peptides may also come from insects and chelicerates and may include but are not limited to cecropins, peneadins, bactenecins, callinectins, myticins, tachyplesins, clavanins, misgurins, pleurocidins, parasins, histones, acidic proteins, and lysozymes. The gene for the chosen protein or peptide is either isolated from the original source, an amplification source, or it can be made synthetically. The gene is spliced into a transformation vector suitable for plant transformation. The transformation vector is chosen so that the antibody will be overexpressed in the plant cellular biomass. The vector may be targeted to the edible portion of the plant (*i*.*e*., seeds) so that normal harvesting methodologies can be used. Alternatively, the vector may be targeted to the unused portion of the plant (stems and leaves) so that these materials can be used as value added components to the crop plant without affecting the yield or quality of the normally harvested portion. This biomass is then used as a feed additive in such a way as to provide the bactericidal protein directly to the animal thus providing resistance to that particular pathogen.

**Example 3. Incorporation of a gene for an antibody or antibody fragment into a plant-based virus and use of the infected plant material as feed.** A particular viral or bacterial pathogen is chosen and used to prepare monoclonal antibodies using procedures described in "Current Protocols in Immunology" or other procedures known to those skilled in this field. Gene(s) coding for this antibody or an appropriate antibody fragment (Fab) are isolated and amplified in the appropriate vector. The gene is spliced into the genome of a selected plant virus such as tobacco mosaic virus (TMV) or cauliflower mosaic virus (CMV). This virus is then used to infect a plant (mature or seedling). As the virus replicates in the plant material it will express the antibody or antibody fragment directly in the plant material. The entire plant can then be harvested and used directly as feed material. Alternatively, the plant material may be homogenized and extruded into pellets suitable for feed applications. The viruses should not be a concern in feeding , since they will not infect the animals consuming the feed, but to the extent there is a concern, they can be inactivated by high temperature or other procedures familiar to those experts in the field prior to use of the plant material as feeds.

**Example 4. Incorporation of a gene for a bactericidal or bacteriostatic protein into a plant-based virus and use of the infected plant material as feed.** A bactericidal or bacteriostatic protein is chosen for the particular application. For example, proteins of the penaeidin class may be chosen for pathogenic control in shrimp. Penaeidins are members of a family of antimicrobial peptides isolated from crustaceans (e.g., Penaeus shrimp). Antimicrobial peptides may also come from insects and chelicerates and may include but are not limited to cecropins, peneadins, bactenecins, callinectins, myticins, tachyplesins, clavanins, misgurins, pleurocidins, parasins, histones, acidic proteins, and lysozymes. The gene for the chosen protein or peptide is either isolated from the original source, an amplification source, or it can be made synthetically. The gene is spliced into the genome of a selected plant virus such as tobacco mosaic virus (TMV) or cauliflower mosaic virus (CMV). This virus is then used to infect a plant (mature or seedling). As the virus replicates in the plant material it will express the protein directly in the plant material. The entire plant can then be harvested and used directly as feed material. Alternatively, the plant material may be homogenized and extruded into pellets suitable for feed applications. The viruses can be inactivated by high temperature or other procedures familiar to those experts in the field prior to use as feeds.

**Example 5. Incorporation of a gene for an antibody or antibody fragment into an insect-based virus and use of the infected insect material as feed.** A particular viral or bacterial pathogen is chosen and used to prepare monoclonal antibodies using procedures well known to experts in this field. Gene(s) coding for this antibody or an appropriate antibody fragment (Fab) are isolated and amplified in the appropriate vector. The gene is spliced into the genome of a selected insect virus such as baculovirus. This virus is then used to infect insect larvae. As the virus replicates in the larval insect will express the antibody or antibody fragment directly in the larval cells. The entire larvae can them be harvested and used directly as feed material. Alternatively, the larvae may be homogenized and extruded into pellets suitable for feed applications. The viruses can be inactivated by high temperature or other procedures familiar to those experts in the field prior to use as feeds.

**Example 6. Incorporation of a gene for a bacteriostatic or bactericidal protein into an insect-based virus and use of the infected insect material as feed.** A bactericidal or bacteriostatic protein is chosen for the particular application. For example, proteins of the penaeidin class may be chosen for pathogenic control in shrimp. Penaeidins are members of a family of antimicrobial peptides isolated from crustaceans (e.g., Penaeus shrimp). Antimicrobial peptides may also come from insects and chelicerates and may include but are not limited to cecropins, peneadins, bactenecins, callinectins, myticins, tachyplesins, clavanins, misgurins, pleurocidins, parasins, histones, acidic proteins, and lysozymes. The gene for the chosen protein or peptide is either isolated from the original source, an amplification source, or it can be made synthetically. The gene is spliced into the genome of a selected insect virus such as baculovirus. This virus is then used to infect insect larvae. As the virus replicates in the larvae it will express the protein directly in the larval tissues. The entire larva can be harvested and used directly as feed material. Alternatively, the larvae may be homogenized and extruded into pellets suitable for feed applications. The viruses can be inactivated by high temperature or other procedures familiar to those experts in the field prior to use as feeds.

**Example 7. Incorporation of a gene for a therapeutic protein into baculovirus and the use of the infected material as feed.** Pacific white shrimp (*Penaeus vannamei*) were transfected orally with an engineered baculovirus (AcNPV-eGFP) to express GFP as a fusion protein. The Bacmid Bac-to-Bac® Baculovirus Expression system (Invitrogen) was utilized for cloning and transfection. A 720 kb fragment containing GFP was fused to the polyhedron (pPolh) promoter and flanked by Xho I sites 3' to pPolh. Using methods described in the invitrogen product literature, Sf9 insect cells were transfected with the recombinant baculovirus. After 72 hours, plaque formation was visually confirmed, and 70 ml culture fluid medium was pelleted at 100 g for 5 minutes at 4°C. The resulting cell pellet was maintained at 4°C until it was subsequently used for oral infection. The corresponding resulting supernatant fluid was centrifuged for 2 hours at 80,000 g at 4°C on a 27% sucrose gradient to yield purified virus. This sucrose-purified virus pellet was maintained at 4°C until it was subsequently used for oral infection.

Shrimp isolation chambers consisting of 3-qt containers filled with 30 ppt salinity dechlorinated water were provided with air stones for oxygenation. Three one-gram shrimp were placed in each container and allowed to acclimatize overnight.

The following procedures were performed within 30 minutes prior to feeding the shrimp. A pellet matrix was prepared by first adding 100 mg of alginic acid (Sigma) to 10 ml of distilled deionized water (ddH₂0) in a beaker and heating to 40 °C while stirring. After the gel began to form, 150 mg of starch (Sigma) was added. The solution was allowed to mix for a minute before addition of 500 mg of krill meal. While continuing to stir the solution, the heat source was removed.

An aliquot of pellet matrix (500 µl) was combined with either 5 µl) of the infected cell pellet or 5 µl of sucrose-purified virus, and gently mixed with vortex mixer. The infected pellet matrix was aspirated into a tuberculin syringe to which a 21-gauge needle was subsequently attached. A formation solution was formed by dissolving 5 grams of calcium chloride (J.T. Baker) and 1 gram of sodium chloride (Research Organics) in 100 ml of ddH₂O. While the formation solution was stirring slowly, the matrix was squeezed through the needle into the solution to form tubular pellets. Pellets formed immediately upon impact in solution and a spatula was used to clean the needle between pellets. Pellets appeared to be 25-30 µl in volume. The pellets were washed in 10% NaCl and added to the shrimp isolation containers. The shrimp immediately consumed the pellets, and were fed to satiation. Each shrimp consumed approximately one pellet.

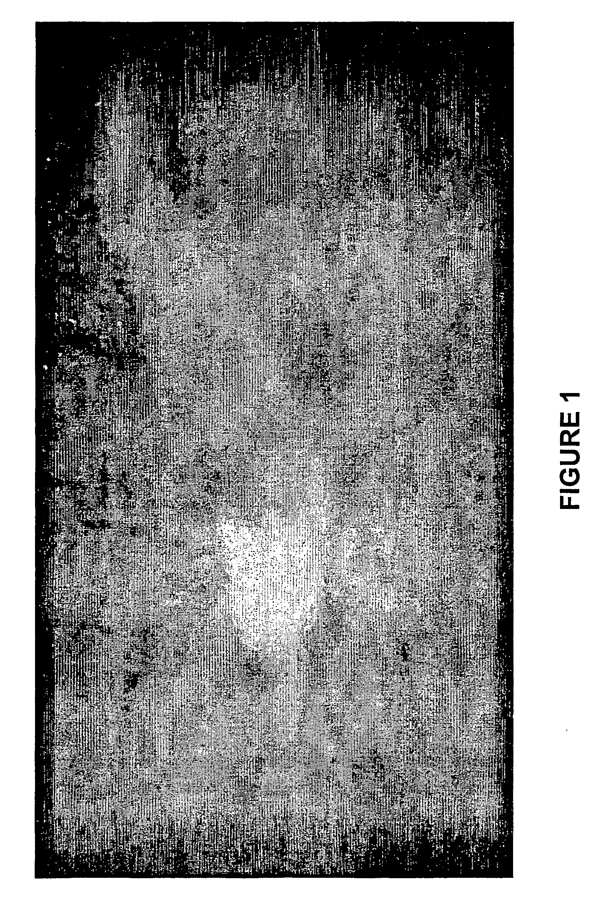

Seventy two hours after consuming the virally infected matrix, the shrimp were placed in a petri dish and observed on a Dark Reader® transilluminator (Claire Chemical Research). Shrimp expressing GFP exhibited a greenish glow (Figure 1). Uninfected shrimp demonstrated no fluorescence. The recombinant GFP-tagged baculovirus observed at 72 h was located specifically within the hepatopancreas area in the cephalothorax (Figure 1).

**Example 8. Vaccination using feeds.** An antigen characteristic to a particular pathogen is chosen as is required by the animal and circumstances. For example, a viral coat protein or component thereof, or an infectious bacterial protein, or a component thereof is chosen. The gene coding for the protein is isolated and incorporated into a vector suitable for use in the plant or insect of choice for production. The transformation vector is chosen so that the protein will be overexpressed in the plant, animal or insect cell biomass or in a virus infecting the plant, animal or insect biomass. This biomass is then used as a feed additive in such a way as to provide the viral or bacterial or fungal protein directly to the animal thus stimulating an immunological response to that particular pathogen. The microbial component may enter the body of the animal in the digestive tract, or otherwise through contact in the air or water.

### References

Ausubel, et al., eds., (1987 and periodic supplements) Current Protocols in Immunology, Wiley-Interscience.
Bac-to-Bac Baculovirus expression systems manual. Invitrogen Life Technologies. Cat. No. 10359-016
Chalfie, M., Tu, Y., Euskirchen, G., Ward, W.W., and Prasher, D.C. (1994) Green Fluorescent Protein as a Marker for Gene Expression. Science. 263: 802-805.
Collagen, et al., eds. (1991 and periodic supplements) Current Protocols in Immunology, Wiley-Interscience.
Inoue, S., and Tsuji, F.I. (1994) Aequorea green-fluorescent protein: Expression of the gene and fluorescence characteristics of the recombinant protein. FEBS Letters. 341: 277-280.
Lewis, David L., De Camillis, Mark A., Brunetti, Craig R., Halder, G., Kassner, Victoria A., Selegue, J.E., Higgs, S., and Carroll, S.B. (1999) Ectopic gene expression and homeotic transformations in arthropods using recombinant Sindbis viruses. Current Biology 9:1279-1287.
Prasher, D.C., Eckenrode, V.K., Ward, W.W., Prendergast, F.G., and Cormier, M.J. (1992) Primary structure of the Aequorea victoria green-fluorescent protein. Gene. 111: 229-233.

## Claims

1. An aquaculture feed containing biomass of an organism selected from plants, animals and insects, wherein said organism is infected with recombinant viruses which encode one or more proteins, antibodies, antibody fragments, or a combination thereof, wherein said proteins and antibodies are expressed recombinantly and non-native to the organism which is the source of the biomass.

2. An agriculture feed containing biomass of an organism selected from plants, animals and insects, wherein said organism is infected with recombinant viruses which encode one or more proteins, antibodies, antibody fragments, or a combination thereof, wherein said proteins and antibodies are expressed recombinantly and non-native to the organism which is the source of the biomass.

3. A human food containing biomass of an organism selected from plants, animals or insects, wherein said organism is infected with recombinant viruses which encode one or more proteins, antibodies, antibody fragments, or a combination thereof, wherein said proteins and antibodies are expressed recombinantly and non-native to the organism which is the source of the biomass.

4. The agricultural feed as claimed in claim 2, or human food as claimed in claim 3, wherein the organism is an agronomic plant selected from tobacco, soybean, corn, sunflower, cotton, safflower, canola and kelp.

5. The aquaculture feed as claimed in claim 1, or agriculture feed as claimed in claim 2, or human food as claimed in claim 3, wherein the therapeutic protein is an antibody specific for bacteria or virus causing disease in the respective aquaculture or agriculture species or human patient.

6. Use of a feed according to claim 1 or 2 for the manufacture of a medicament as therapeutic proteins for non-human animal.

7. Use according to claim 6, wherein the non-human animal is subjected to intensive agricultural practices.

8. Use according to claim 6, wherein the non-human animal comprises fish or shellfish in aquaculture.

9. Use according to claim 6, wherein the organism is infected by a recombinant virus which encodes the therapeutic protein and the therapeutic protein is expressed recombinantly.

10. Use according to claim 6, wherein the therapeutic protein is a protein which inhibits growth or replication of *Vibrio* species *in vitro.*

11. Use according to claim 6, wherein the therapeutic protein is a protein which inhibits Taura or White spot virus infection in shrimp.

12. Use according to claim 6, wherein the therapeutic protein is a recombinantly expressed antibody or fragment thereof.

13. Use according to claim 6, wherein the recombinantly expressed antibody or fragment thereof specifically binds to an infectious agent of disease in the non-human animal.

14. Use of a recombinant Autographa californica nuclear polyhedrosis virus for the manufacture of a medicament as therapeutic proteins for crustaceans edible material containing said virus.

15. Use according to claim 14, wherein the source of said virus is the supernatant of cultured cells infected with said virus.

16. Use according to claim 14, wherein the source of said virus is cultured cells infected with said virus.

## Patentansprüche

1. Aquakultur-Futtermittel, welches Biomasse eines Organismus, ausgewählt aus Pflanzen, Tieren und Insekten, enthält, wobei der Organismus mit rekombinanten Viren, die ein oder mehrere Proteine, Antikörper, Antikörperfragmente oder eine Kombination davon kodieren, infiziert ist, wobei die Proteine und Antikörper rekombinant exprimiert werden und zu dem Organismus, der die Quelle der Biomasse ist, nicht-nativ sind.

2. Landwirtschaftliches Futtermittel, welches Biomasse eines Organismus, ausgewählt aus Pflanzen, Tieren und Insekten, enthält, wobei der Organismus mit rekombinanten Viren, die ein oder mehrere Proteine, Antikörper, Antikörperfragmente oder eine Kombination davon kodieren, infiziert ist, wobei die Proteine und Antikörper rekombinant exprimiert werden und zu dem Organismus, der die Quelle der Biomasse ist, nicht-nativ sind.

3. Für den Menschen bestimmtes Nahrungsmittel, welches Biomasse eines Organismus, ausgewählt aus Pflanzen, Tieren oder Insekten, enthält, wobei der Organismus mit rekombinanten Viren; die ein oder mehrere Proteine, Antikörper, Antikörperfragmente oder eine Kombination davon kodieren, infiziert ist, wobei die Proteine und Antikörper rekombinant exprimiert werden und zu dem Organismus, der die Quelle der Biomasse ist, nicht-nativ sind.

4. Landwirtschaftliches Futtermittel nach Anspruch 2 oder für den Menschen bestimmtes Nahrungsmittel nach Anspruch 3, wobei der Organismus eine landwirtschaftliche Pflanze ist, welche aus Tabak, Sojabohne, Getreide, Sonnenblume, Baumwolle, Saflor, Raps und Seetang ausgewählt ist.

5. Aquakultur-Futtermittel nach Anspruch 1 oder landwirtschaftliches Futtermittel nach Anspruch 2 oder für den Menschen bestimmtes Nahrungsmittel nach Anspruch 3, wobei das therapeutische Protein ein Antikörper ist, der für Bakterien oder Viren, die in der jeweiligen Aquakultur oder landwirtschaftlichen Spezies oder dem jeweiligen humanen Patienten Krankheiten verursachen, spezifisch ist.

6. Verwendung eines Futtermittels nach Anspruch 1 oder 2 zur Herstellung eines Arzneimittels als therapeutische Proteine für ein nicht-menschliches Tier.

7. Verwendung nach Anspruch 6, wobei das nicht-menschliche Tier intensiven landwirtschaftlichen Praktiken unterzogen wird.

8. Verwendung nach Anspruch 6, wobei das nicht-menschliche Tier Fische oder Schalentiere in Aquakultur umfasst.

9. Verwendung nach Anspruch 6, wobei der Organismus durch ein rekombinantes Virus, welches das therapeutische Protein kodiert, infiziert ist und das therapeutische Protein rekombinant exprimiert wird.

10. Verwendung nach Anspruch 6, wobei das therapeutische Protein ein Protein ist, welches die Vermehrung oder Replikation von Vibrio-Spezies *in vitro* inhibiert.

11. Verwendung nach Anspruch 6, wobei das therapeutische Protein ein Protein ist, welches in Shrimps eine Taura- oder Wieße Punkte-Virus-Infektion inhibiert.

12. Verwendung nach Anspruch 6, wobei das therapeutische Protein ein rekombinant exprimierter Antikörper oder ein Fragment davon ist.

13. Verwendung nach Anspruch 6, wobei der rekombinant exprimierte Antikörper oder das Fragment davon spezifisch an einen infektiösen Krankheitserreger in dem nicht-menschlichen Tier bindet.

14. Verwendung eines rekombinanten nukleären Polyhedrose-Virus aus Autographa californica zur Herstellung eines Arzneimittels als therapeutische Proteine für ein essbares Krustentier-Material, welches das Virus enthält.

15. Verwendung nach Anspruch 14, wobei die Quelle des Virus der Überstand von kultivierten Zellen, die mit dem Virus infiziert sind, ist.

16. Verwendung nach Anspruch 14, wobei die Quelle des Virus kultivierte Zellen, die mit dem Virus infiziert sind, ist.

## Revendications

1. Aliment aquacole contenant de la biomasse d'un organisme sélectionné parmi les plantes, les animaux et les insectes, dans lequel ledit organisme est infecté par des virus recombinants qui codent une ou plusieurs protéines, anticorps, fragments d'anticorps, ou combinaison de ceux-ci, dans lequel lesdites protéines et lesdits anticorps sont exprimés de manière recombinante et non native en direction de l'organisme qui est la source de la biomasse.

2. Aliment agricole contenant de la biomasse d'un organisme sélectionné parmi les plantes, les animaux et les insectes, dans lequel ledit organisme est infecté par des virus recombinants qui codent une ou plusieurs protéines, anticorps, fragments d'anticorps, ou combinaison de ceux-ci, dans lequel lesdites protéines et lesdits anticorps sont exprimés de manière recombinante et non native en direction de l'organisme qui est la source de la biomasse.

3. Aliment destiné à l'être humain contenant de la biomasse d'un organisme sélectionné parmi les plantes, les animaux et les insectes, dans lequel ledit organisme est infecté par des virus recombinants qui codent un ou une ou plusieurs protéines, anticorps, fragments d'anticorps, ou combinaison de ceux-ci, dans lequel lesdites protéines et lesdits anticorps sont exprimés de manière recombinante et non native en direction de l'organisme qui est la source de la biomasse.

4. Aliment agricole selon la revendication 2, ou aliment destiné à l'être humain selon la revendication 3, dans lequel l'organisme est une plante agronomique choisie parmi le tabac, le soja, le maïs, le tournesol, le coton, le carthame, le canola et les algues géantes (kelp).

5. Aliment aquacole selon la revendication 1, ou aliment agricole selon la revendication 2, ou aliment destiné à l'être humain selon la revendication 3, dans lequel la protéine thérapeutique est un anticorps spécifique pour une bactérie ou un virus provoquant une maladie dans les espèces aquacoles ou agricoles respectives ou chez un patient humain.

6. Utilisation d'un aliment selon la revendication 1 ou 2 pour la fabrication d'un médicament sous forme de protéines thérapeutiques pour un animal non humain.

7. Utilisation selon la revendication 6, dans laquelle l'animal non humain est soumis à des pratiques agricoles intensives.

8. Utilisation selon la revendication 6, dans laquelle l'animal non humain comprend un poisson ou un crustacé ou coquillage en aquaculture.

9. Utilisation selon la revendication 6, dans laquelle l'organisme est infecté par un virus recombinant qui code la protéine thérapeutique et la protéine thérapeutique est exprimée de manière recombinante.

10. Utilisation selon la revendication 6, dans laquelle la protéine thérapeutique est une protéine qui inhibe la croissance ou la réplication des espèces *Vibrio in vitro.*

11. Utilisation selon la revendication 6, dans laquelle la protéine thérapeutique est une protéine qui inhibe l'infection par le virus du syndrome de Taura ou le virus du syndrome des points blancs chez les crevettes.

12. Utilisation selon la revendication 6, dans laquelle la protéine thérapeutique est un anticorps ou un fragment de celui-ci exprimé de manière recombinante.

13. Utilisation selon la revendication 6, dans laquelle l'anticorps ou un fragment de celui-ci exprimé de manière recombinante se lie spécifiquement à un agent infectieux de la maladie chez l'animal non humain.

14. Utilisation d'un virus recombinant de la polyhédrose nucléaire *Autographa californica* pour la fabrication d'un médicament sous la forme de protéines thérapeutiques pour la chair comestible des crustacés contenant ledit virus.

15. Utilisation selon la revendication 14, dans laquelle la source dudit virus est le surnageant de cellules cultivées infectées par ledit virus.

16. Utilisation selon la revendication 14, dans laquelle la source dudit virus est constituée par des cellules cultivées infectées par ledit virus.
